# EUROPÄISCHE PATENTSCHRIFT

(11) **EP 0 768 863 B1**
(45) Veröffentlichungstag und Bekanntmachung des Hinweises auf die Patenterteilung: **06.10.1999**
(21) Anmeldenummer: 95924275.1
(22) Anmeldetag: 19.06.1995
(51) Int. Cl.: A61K 7/06, A61K 7/50, A61K 7/48

(54) **KOSMETISCHE MITTEL**
COSMETICS AGENTS
AGENTS COSMETIQUES

(30) Priorität: 27.06.1994 DE 4422404
(43) Veröffentlichungstag der Anmeldung: 23.04.1997
(73) Patentinhaber: Henkel Kommanditgesellschaft auf Aktien, 40191 Düsseldorf (DE)
(72) Erfinder: FABRY, Bernd, D-41352 Korschenbroich (DE)
(86) Internationale Anmeldenummer: EP9502360
(87) Internationale Veröffentlichungsnummer: WO9600054

(56) Entgegenhaltungen:
- EP-A- 0 416 447
- EP-A- 0 422 508
- DE-A- 4 335 782
- DATABASE WPI Section Ch, Week 9351 Derwent Publications Ltd., London, GB; Class A61,Page 1, AN 93-410769 & JP,A,05 310 542 (KAO CORP.)

## Beschreibung

Die Erfindung betrifft kosmetische Mittel mit einem Gehalt an Fettsäure-N-alkylpolyhydroxyalkylamiden und kationischen Polymeren.

### Stand der Technik

Fettsäure-N-alkylpolyhydroxyalkylamide und insbesondere Fettsäure-N-alkylglucamide stellen bekannte nichtionische Tenside auf Zuckerbasis dar, die sich durch ein starkes Schaumvermögen, gute Reinigungsleistung und hohe hautkosmetische Verträglichkeit auszeichnen.

Die Verwendung dieser Stoffe ist Gegenstand einer Vielzahl von Veröffentlichungen. Aus der Europäischen Patentanmeldung **EP-A1 0285768** (Hüls) ist beispielsweise ihr Einsatz als Verdickungsmittel bekannt. In der Französischen Offenlegungsschrift **FR-A 1580491** (Henkel) werden wäßrige Detergensgemische auf Basis von Sulfaten und/oder Sulfonaten, Niotensiden und gegebenenfalls Seifen beschrieben, die Fettsäure-N-alkylglucamide als Schaumregulatoren enthalten.

Gegenstand der Internationalen Patentanmeldungen **WO 92/6153; 6156; 6157; 6158; 6159** und **6160** (Procter & Gamble) sind Mischungen von Fettsäure-N-alkylglucamiden mit anionischen Tensiden, Tensiden mit Sulfat- und/oder Sulfonatstruktur, Ethercarbonsäuren, Ethersulfaten, Methylestersulfonaten und nichtionischen Tensiden. Die Verwendung dieser Stoffe in den unterschiedlichsten Wasch-, Spül- und Reinigungsmitteln wird in den Internationalen Patentanmeldungen **WO 92/6152; 6154; 6155; 6161; 6162; 6164; 6170; 6171** und **6172** (Procter & Gamble) beschrieben.

Hinsichtlich der Formulierung von kosmetischen Mitteln, insbesondere Haarpflegemitteln, weisen Fettsäure-N-alkylpolyhydroxyalkylamide Jedoch den Nachteil auf, daß ihr Schaumvermögen nicht immer zufriedenstellend ist. Dies bezieht sich einerseits auf die Höhe des Basischaums, andererseits auch auf die Schaumbeständigkeit, insbesondere in hartem Wasser. Ein weiterer Nachteil besteht darin, daß die Amide nach dem Abspülen oftmals ein stumpfes Gefühl auf den Haaren hinterlassen und die Kämmbarkeit tendenziell eher verschlechtern.

Die komplexe Aufgabe der Erfindung hat somit darin bestanden, kosmetische Mittel mit einem Gehalt an Fettsäure-N-alkylpolyhydroxyalkylamiden zur Verfügung zu stellen, die frei von den geschilderten Nachteilen sind.

### Beschreibung der Erfindung

Gegenstand der Erfindung sind kosmetische Mittel, enthaltend
(a) Fettsäure-N-alkylpolyhydroxyalkylamide und
(b) kationische Polymere.
mit der Maßgabe, daß sie frei sind von Harnstoff und Hydroxy-4-methyl-6-(2,4,4-trimethylpentyl)-2-pyridon-Monoethanolamin-Salz.

Überraschend wurde gefunden, daß der Zusatz von kationischen Polymeren das Schaumvermögen von Fettsäure-N-alkylpolyhydroxyalkylamiden und insbesondere Fettsäure-N-methylglucamiden signifikant steigert. Die Erfindung schließt die Erkenntnis ein, daß in gleicher Weise die Schaumbeschaffenheit und die Haaravivage verbessert werden.

### Fettsäure-N-alkylpolyhydroxyalkylamide

Fettsäure-N-alkylpolyhydroxyalkylamide folgen der Formel **(I)**, 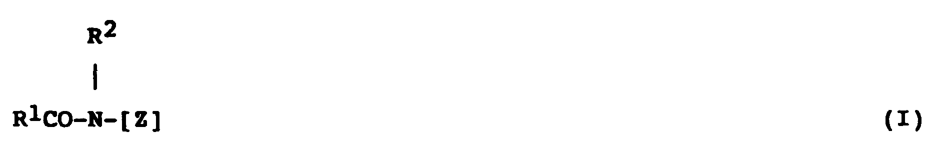 in der R¹CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1985424, US 2016962** und **US 2703798** sowie die Internationale Patentanmeldung **WO 92/06984** verwiesen. Eine Übersicht zu diesem Thema von H.Kelkenberg findet sich in **Tens. Surf.Det. 25, 8 (1988).**

Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(Ia)** wiedergegeben werden:

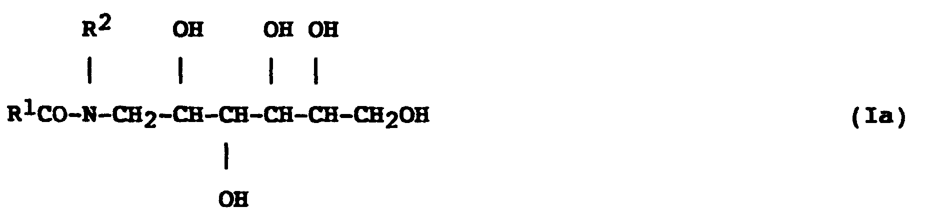

Vorzugsweise werden als Fettsäure-N-alkylpolyhydroxyalkylamide Glucamide der Formel **(Ia)** eingesetzt, in der R² für Wasserstoff oder eine Amingruppe steht und R¹CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(Ia)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

### Kationische Polymere

Kationische Polymere stellen bekannte Stoffe dar, die nach den einschlägigen Verfahren der technischen organischen Chemie erhalten werden können. Typische Beispiele für kationische Polymere, die im Sinne der Erfindung als Komponente (b) in Betracht kommen, sind:
(b1) **Kationische Cellulosederivate,** die der Formel **(II)** folgen, 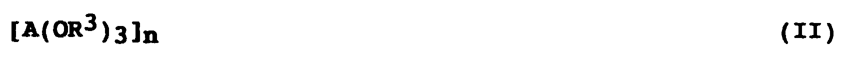 in der A für einen Anhydroglucoserest, n für Zahlen von 50 bis 20.000 und R² für einen Rest der Formel **(III)** steht, 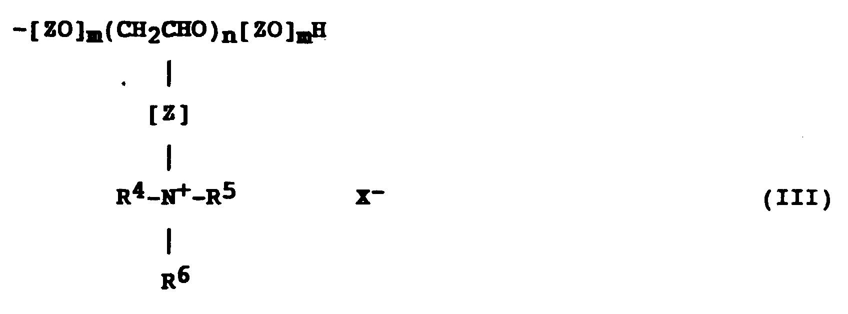 in der Z für gegebenenfalls hydroxysubstituierte Alkylengruppen mit 2 oder 3 Kohlenstoffatomen, m für Zahlen von 0 bis 10, n für Zahlen von 0,001 bis 3 und R⁴, R⁵ und R⁶ unabhängig voneinander für Alkyl-, Aryl- oder Aralkylgruppen mit 1 bis 10 Kohlenstoffatomen stehen oder einen heterocyclischen Ring mit dem Stickstoffatom bilden, und X schließlich ein Anion aus der Gruppe bedeutet, die gebildet wird von Halogen, Sulfat, Sulfonat, Methosulfat oder Phosphat.
   Der kationische Substitutionsgrad der in Betracht kommenden kationischen Cellulosen - entsprechend dem Parameter "n" in Formel **(III)** - liegt vorzugsweise im Bereich von 0,01 bis 1 und beträgt insbesondere 0,02 bis 0,5. Das Molekulargewicht der kationischen Cellulosen liegt ferner vorzugsweise im Bereich von 100.000 bis 3.000.000.
(b2) **Kationische Stärken** der Formel **(IV)**, 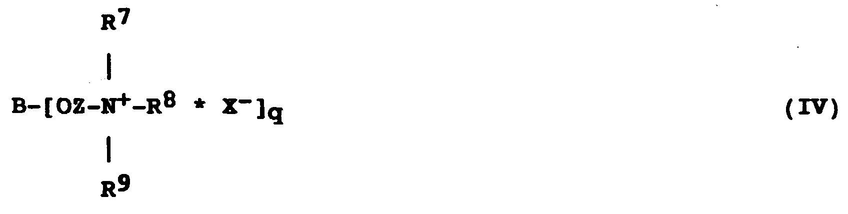 in der B für einen Stärkerest, q für Zahlen von 0,001 bis 1, Z für eine gegebenenfalls hydroxysubstituierte Hydroxyalkylengruppe mit 2 bis 3 Kohlenstoffatomen, R⁷, R⁸ und R⁹ unabhängig voneinander für Alkyl-, Aryl- oder Aralkylgruppen mit 1 bis 10 Kohlenstoffatomen stehen oder einen heterocyclischen Ring mit dem Stickstoffatom bilden, und X schließlich ein Anion aus der Gruppe bedeutet, die gebildet wird von Halogen, Sulfat, Sulfonat, Methosulfat oder Phosphat.
   Kationische Stärken, die im Sinne der Erfindung bevorzugt sind, lassen sich beispielsweise durch alkalikatalysierte Veretherung von Stärke mit Reagenzien, die tertiäre Amino- oder quaternäre Ammoniumgruppen enthalten, als da beispielsweise sind: (2-Chlorethyl)-diethylamin, (2,3-Epoxpropyl)diethylamin, (3-Chlorpropyl)trimethylammoniumchlorid, (3-Chlor-2-hydroxypropyl)trimethylammoniumchlorid, (2,3-Epoxypropyl)trimethylammoniumbromid und (4-Chlor-2-butenyl)trimethylammoniumchlorid. Weite geeignete kationischen Stärken lassen sich auch durch Umsetzung von Stärke mit Cyanamiden herstellen.
   Der kationische Substitutionsgrad, d.h. die Anzahl kationischer Gruppen pro Anhydroglucoseeinheit beträgt vorzugsweise 0,01 bis 1 und insbeson- dere 0,02 bis 0,5.
(b3) **Copolymere von Diallylammoniumsalzen und Acrylamiden** gemäß den Formeln **(Va)** und **(Vb),** 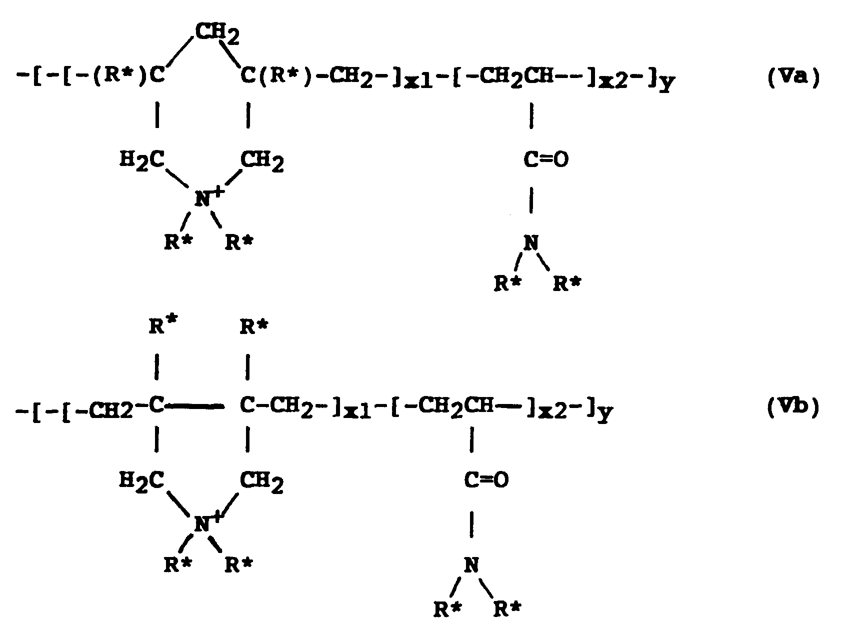 in denen R* jeweils für Wasserstoff oder gegebenenfalls hydroxysubstituierte Alkyl- und/oder Alkenylreste mit 1 bis 18, vorzugsweise 1 bis 12 und insbesondere 1 bis 4 Kohlenstoffatomen, x1 und x2 unabhängig voneinander für Zahlen von 1 bis 50, y für Zahlen von 150 bis 2000 und X für ein Anion aus der Gruppe steht, die gebildet wird von Halogen, Sulfat, Sulfonat, Methosulfat oder Phosphat. Das Molekulargewicht der kationischen Copolymeren kann vorzugsweise im Bereich von 30.000 bis 2.000.000 und insbesondere 100.000 bis 1.000.000 liegen.
(b4) **Quaternierte Polyvinylpyrrolidon-Derivate,** die als Wiederholungseinheit Monomere der Formel **(VI)** enthalten, 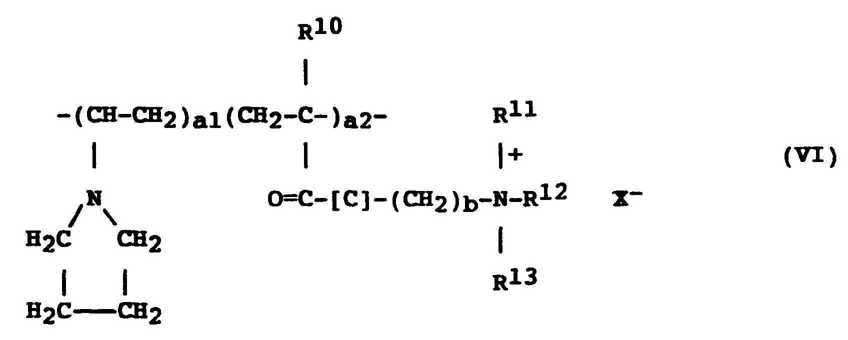 in der R¹⁷ für Wasserstoff oder einen Alkylrest mit 1 bis 4 Kohlenstoffatomen, R¹¹, R¹² und R¹³ unabhängig voneinander für Wasserstoff, gegebenenfalls hydroxysubstituierte Alkyl- und/oder Alkenyl-, Amidoalkyl-, Cyanoalkyl-, Alkoxyalkyl- oder Carbalkoxyalkylgruppen mit 1 bis 18 Kohlenstoffatomen, B für Sauerstoff oder eine NH-Gruppe, die Summe (a1 + a2) für Zahlen von 20 bis 10.000, b für Zahlen von 1 bis 10 und X für ein Anion aus der Gruppe steht, die gebildet wird von Halogen, Sulfat, Sulfonat, Methosulfat oder Phosphat.
   Das Molekulargewicht der kationischen Polymeren kann ebenfalls im Bereich von 30.000 bis 2.000.000 und insbesondere 100.000 bis 1.000.000 liegen. Der Gehalt an kationischem Stickstoff in den Polieren der Formel **(VI)** kann ferner 0,004 bis 0,2, vorzugsweise 0,1 bis 0,15 betragen.

### Als weitere kationische Polymere kommen in Betracht:

- ***: quaternierte Vinylpyrrolidon Vinylimidazol-Polymere wie z.B. LUVICUAT (BASF AG, Ludwidshaften/FRG);
- ***: Kondensationsprodukte von Polyglycolen und Aminen;
- ***: quaternierte Kollagenpolypeptide wie beispielsweise Lauryldimonium hydroxypropyl hydrolyzed collagen (Lamequat^{(R)} L, Grünau GmbH).
- ***: Polyethylenimin;
- ***: kationische Siliconpolymere wie z.B. Amidomethicone oder DOW CORNING 929, Dow Corning Co./US;
- ***: Copolymere der Adipinsäure und Dimethylaminohydroxypropyldiethylentrimamin (CARTARETINE^{(R)}, Sandoz/CH);
- ***: Polyaminopolyamide wie z.B. beschrieben in der **FR-A 2252840** sowie deren vernetzte wasserlöslichen Polymere;
- ***: kationische Chitinderivate wie beispielsweise quaterniertes Chitosan, gegebenenfalls mikrokristallin verteilt:
- ***: kationischer Guar-Gum wie z.B. JAGUAR^{(R)} C-B-S, JAGUAR^{(R)} C-17, JAGUAR^{(R)} C-16 der Celanese/US;
- ***: quaternierte Ammoniumsalz-Polymere wie z.B. MIRAPOL^{(R)} A-15, MIRAPOL^{(R)} AD-1, MIRAPOL^{(R)} AZ-1 der Miranol/US.

Weitere kationische Polymere, die ebenfalls im Sinne der Erfindung in Betracht gezogen werden können, werden in den folgenden Schriften beschrieben, auf deren Lehren hiermit ausdrücklich Bezug genommen wird: **US 2261002, US 2271378, US** **2273780, US 2388614, US 2454547, US 2961347, US 3206462** und **US 3227615.**

Die kationischen Polymeren können in den erfindungsgemäßen Mittel in Mengen von 0,1 bis 5, vorzugsweise 0,2 bis 0,7 Gew.-% - bezogen auf die Mittel - enthalten sein.

### Kosmetische Mittel

Bei der erfindungsgemäßen Mitteln handelt es sich vorzugsweise um haarkosmetische Produkte, also beispielsweise Haarshampoos, Haarspülungen, Haarkuren und dergleichen. Gemische von Fettsäure-N-alkylpolyhydroxyalkylamiden und kationischen Polymeren können jedoch in gleicher Weise auch zur vorteilhaften Herstellung von Körperreinigungsmitteln bzw. Produkten zur Hautpflege wie beispielsweise Duschgels oder Schaumbädern eingesetzt werden.

Die Mittel können weitere, mit den anderen Inhaltsstoffen kompatible **Tenside** enthalten. Typische Beispiele sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Ethercarbonsäuren, Alkyl- und/oder Alkenyloligoglykoside, Alkylamidobetaine oder Eiweißfettsäurekondensate. Besonders bevorzugt sind Kombinationen von Fettsäure-N-alkylglucamiden, Eiweißfettsäurekondensaten und Betainen.

Als Hilfs- und Zusatzstoffe kommen fermer auch **Emulgatoren** wie etwa alkoxylierte Fettalkohole oder Sorbitanester in Betracht. Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid. Unter **biogenen Wirkstoffen** sind beispielsweise Pflanzenextrakte, Eiweißhydrolysate und Vitaminkomplexe zu verstehen. Gebräuchliche Filmbildner sind beispielsweise Chitosan, mikrokristallines Chitosan und ähnliche Verbindungen. Als **Konservierungsmittel** eignen sich beispielsweise Phenoxyethanol, Formaldehydlösung, Parabene, Pentadiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Üblicherweise liegen die Mittel in wäßriger Form vor. Dabei kann der Aktivsubstanzgehalt im Bereich von 3 bis 50 und vorzugsweise bei 10 bis 25 Gew.-% liegen.

### Gewerbliche Anwendbarkeit

Mischungen von Fettsäure-N-alkylpolyhydroxyalkylamiden und kationischen Polymeren sind mild und zeichnen sich durch ein verbessertes Schaumvermögen sowie eine verbesserte Haaravivage aus. Sie eignen sich daher zur Herstellung von kosmetischen Mitteln, insbesondere haarkosmetischen Mitteln wie beispielsweise Haarshampoos, Haarspülungen und Haarkuren.

Die folgenden Beispiele sollen den Gegenstand der Erfindung näher erläutern, ohne ihn darauf einzuschränken.

### Beispiele

### Ia. Eingesetzte Tenside

A1) C_{12/14}-Kokosalkyl-N-methylglucamid
A2) Lauryl-N-methylglucamid
B1) C_{12/14}-Kokosalkohol-3,6EO-sulfat-Na-Salz
B2) C_{8/10}-Alkylpolyglucosid, DP = 1,5
B3) Sojaproteinkokosfettsäurekondensat
B4) Betaine auf Basis Kokosfettsäure

### Ib. Eingesetzte Kationpolymere

K1) LAMEQUAT^{(R)} L, Lauryldimonium hydroxypropyl hydrolyzed collagen, Grünau;
K2) Polymer JR-400, Union Carbide; kationisierte Cellulose;
K3) MERQUAt^{(R)} 550, Copolymer auf Basis von N,N-dimethyl-1,3-methylenpiperidinium chlorid, Merck Co.;
K4) GAFQUAT^{(R)} 755, Copolymer auf Basis Vinylpyrrolidon und Dimethylaminoethylacrylat, GAF Chemical Co.

### Ic. Zusatzstoffe

Z1) 2-Octyldodecanol
Z2) Dioctylcyclohexan
Z3) Mandelöl
Z4) Di-n-octylether
Z5) Cetearylalkohol

### II. Rezepturen

Die Rezepturen R1 bis R4 sind erfindungsgemäß, die Rezepturen R5 bis R8 dienen dem Vergleich. Die Angaben in Tabelle verstehen sich als Gew.-% (Wasser ad 100 Gew.-%).

**Tabelle 1**

| Zusammensetzung der untersuchten Rezepturen | | | | | | | | |
|---|---|---|---|---|---|---|---|---|
| **Komponente** | **Rezepturen** | | | | | | | |
| | **R1** | **R2** | **R3** | **R4** | **R5** | **R6** | **R7** | **R8** |
| A1 | 4 | - | 2 | - | 4 | - | - | - |
| A2 | - | 4 | - | 2 | - | 2 | - | - |
| B1 | 8 | 8 | 6 | 6 | 8 | 6 | 8 | 6 |
| B2 | - | - | - | - | - | - | 4 | 2 |
| B3 | - | - | 2 | 2 | - | 2 | - | 2 |
| B4 | - | - | 2 | 2 | - | 2 | - | 2 |
| K1 | 1 | - | - | - | - | - | 1 | - |
| K2 | - | 1 | - | - | - | - | - | - |
| K3 | - | - | 1 | - | - | - | - | - |
| K4 | - | - | - | 1 | - | - | - | 1 |
| Z1 | 2 | - | - | - | 2 | - | 2 | - |
| Z2 | - | 2 | - | - | - | - | - | 2 |
| Z3 | - | - | 2 | - | - | 2 | - | - |
| Z4 | - | - | - | 2 | - | - | - | - |
| Z5 | 3 | 3 | 3 | 3 | 3 | 3 | 3 | 3 |

### III. Schaumvermögen

Das Schaumvermögen der Rezepturen wurde in hartem Wasser (16 d) bei 23°C gemäß DIN 53 902/Teil 2 bestimmt. Angegeben sind der Basisschaum, die Schaumhöhe nach 5 min, die Beschaffenheit des Schaums sowie das subjektive Haargefühl bei Anwendung als Haarshampoo nach dem Ausspülen. Die Ergebnisse sind in Tabelle 2 zusammengefaßt.

**Tabelle 2**

| Schaumvermögen | | | | | | | | |
|---|---|---|---|---|---|---|---|---|
| **Parameter** | **Erfgem. Rezepturen** | | | | **Vergleichsrezepturen** | | | |
| | **R1** | **R2** | **R3** | **R4** | **R5** | **R6** | **R7** | **R8** |
| BS (ml) | 600 | 580 | 650 | 630 | 550 | 600 | 590 | 640 |
| SZ (ml) | 550 | 520 | 600 | 580 | 490 | 550 | 550 | 610 |
| SP | ++ | ++ | ++ | ++ | + | + | ++ | ++ |
| HG | +++ | +++ | +++ | +++ | ++ | ++ | + | + |
| Legende: BS = Basisschaum SZ = Schaumzerfall, Schaumhöhe nach 5 min SP = Schaumperformance ++ cremiger, ergiebiger Schaum + grobporiger Schaum HG = Subjektives Haargefühl nach Ausspülen +++ glatt , leicht kämmbar ++ stumpf, leicht kämmbar + stumpf, schwer kämmbar | | | | | | | | |

### IV. Trocken- und Naßkämmbarkeitsuntersuchungen

IVa) Trockenkämmbarkeit
   Die Trockenkämmbarkeit wurde unter Zulassung der elektrostatischen Aufladung untersucht. Es wurde eine relative Luftfeuchtigkeit von 20 % eingestellt. Die Konditionierungszeit betrug 12 h bei 30°C. Die Messung erfolgte über den Ladungsabgriff an einem doppelten Faraday-Käfig nach Ausführung von 10 Kämmungen. Der Fehler bei den Messungen betrug im Mittel 2,5 %, die statistische Sicherheit lag bei mindestens 99,9 %. Die Ergebnisse der Kämmarbeiten sind in Tabelle 3 dargestellt.
IVb) Naßkämmbarkeit
   Die Naßkämmbarkeit wurde an braunem Haar (Alkinco #6634, Strähnenlänge 12 cm, Strähnenmasse 1 g) untersucht. Nach der Nullmessung wurden die Strähnen mit 100 ml der Formulierungen A bis J bzw. U, V und W getränkt. Nach einer Einwirkzeit von 5 min wurden die Strähnen 1 min unter fließendem Wasser (1 l/min, 38°C) ausgespült. Die Strähnen wurden erneut vermessen und mit der Nullmessung verglichen. Der Fehler bei den Messungen betrug im Mittel 2 %, die statistische Sicherheit lag bei mindestens 99 %. Die Ergebnisse sind ebenfalls in Tabelle 3 dargestellt.

**Tabelle 3**

| Trocken- und Naßkämmbarkeit | | | | | | | |
|---|---|---|---|---|---|---|---|
| **Bsp.** | **R** | **Trockenkämmbarkeit [mJ]** | | | **Naßkämmbarkeit [mJ]** | | |
| | | **vor.** | **nach.** | **Diff.** | **vor.** | **nach.** | **Diff.** |
| 1 | 1 | 4,7 | 4,0 | 0,7 | 55,5 | 10,1 | 44,4 |
| 2 | 2 | 4,8 | 4,3 | 0,5 | 55,3 | 11,5 | 43,8 |
| 3 | 3 | 5,2 | 4,2 | 1,0 | 76,5 | 17,5 | 59,0 |
| 4 | 4 | 4,7 | 4,0 | 0,7 | 65,1 | 13,4 | 51,7 |
| V1 | 5 | 4,0 | 3,9 | 0,1 | 46,8 | 17,8 | 29,0 |
| V2 | 6 | 4,2 | 4,2 | 0,2 | 43,6 | 14,9 | 28,7 |
| V3 | 7 | 4,4 | 4,1 | 0,3 | 48,4 | 11,0 | 37,4 |
| V4 | 8 | 4,4 | 4,1 | 0,3 | 51,6 | 15,8 | 35,8 |
| Legende: R = Rezeptur vor. = vorher nach. = nachher Diff. = Differenz | | | | | | | |

## Patentansprüche

1. Kosmetische Mittel, enthaltend
(a) Fettsäure-N-alkylpolyhydroxyalkylamide und
(b) kationische Polymere,
mit der Maßgabe, daß sie frei sind von Harnstoff und Hydroxy-4-methyl-6-(2,4,4-trimethylpentyl)-2-pyridon-Monoethanolamin-Salz.

2. Kosmetische Mittel nach Anspruch 1, **dadurch gekennzeichnet**, daß sie Fettsäure-N-alkylpolyhydroryalkylamide der Formel **(I)** enthalten, 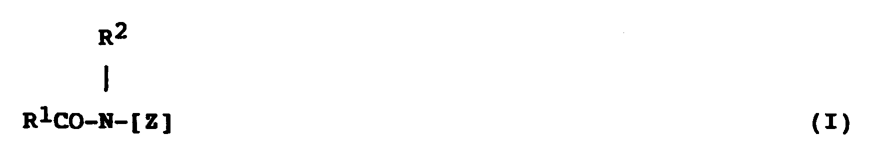 in der R¹CO für einen aliphatischen Acylrest mit 6 bis 22 Kohlenstoffatomen, R² für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

3. Kosmetische Mittel nach den Ansprüchen 1 und 2, **dadurch genennzeichnet**, daß sie als Fettsäure-N-alkylpolyhydroxyalkylamide Fettsäure-N-methylglucamid enthalten.

4. Kosmetische Mittel nach den Ansprüchen 1 und 2, **dadurch genennzeichnet**, daß sie die Fettsäure-N-alkylpolyhydroxyalkylamide in Mengen von 0,5 bis 25 Gew.-% - bezogen auf die Mittel - enthalten.

5. Kosmetische Mittel nach den Ansprüchen 1 bis 4, **dadurch genennzeichnet**, daß sie kationische Polymere enthalten, die ausgewählt sind aus der Gruppe, die gebildet wird von kationischen Cellulosederivaten, kationischen Stärken, Copolymeren von Diallylammoniumsalzen und Acrylamiden, quaternierten Polyvinylpyrrolidon-Derivaten, quaternierten Vinylpyrrolidon Vinylimidazol-Polymeren, Kondensationsprodukten von Polyglycolen und Aminen, quaternierten Kollagenpolypeptiden, Polyethylenimin, kationischen Siliconpolymeren, Copolymeren der Adipinsäure und Dimethylaminohydroxypropyldiethylentrimamin, Polyaminopolyamiden, kationischen Chitinderivaten, kationischem Guar-Gum und/oder quaternierten Ammoniumsalz-Polymeren.

6. Kosmetische Mittel nach den Ansprüchen 1 bis 5, **dadurch genennzeichnet**, daß sie die kationischen Polymeren in Mengen von 0,1 bis 5 Gew.-% enthalten.

## Claims

1. Cosmetic compositions containing
(a) fatty acid-N-alkyl polhydroxyalkyl amides and
(b) cationic polymers,
with the proviso that they are free from urea and hydroxy-4-methyl-6-(2,4,4-trimethylpentyl)-2-pyridone monoethanolamine salt.

2. Cosmetic compositions as claimed in claim 1, characterized in that they contain fatty acid-N-alkyl polyhydroxyalkyl amides corresponding to formula (I): 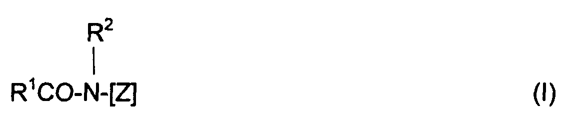 in which R¹CO is an aliphatic acyl group containing 6 to 22 carbon atoms, R² is hydrogen, an alkyl or hydroxyalkyl group containing 1 to 4 carbon atoms and [Z] is a linear or branched polyhydroxyalkyl group containing 3 to 12 carbon atoms and 3 to 10 hydroxyl groups.

3. Cosmetic compositions as claimed in claims 1 and 2, characterized in that they contain fatty acid-N-methyl glucamide as the fatty acid-N-alkylpolyhydroxyalkyl amide.

4. Cosmetic compositions as claimed in claims 1 and 2, characterized in that they contain the fatty acid-N-alkyl polyhydroxyalkyl amides in quantities of 0.5 to 25% by weight, based on the composition.

5. Cosmetic compositions as claimed in claims 1 to 4, characterized in that they contain cationic polymers selected from the group consisting of cationic cellulose derivatives, cationic starches, copolymers of diallylammonium salts and acrylamides, quaternized vinyl pyrrolidone/vinyl imidazole polymers, condensates of polyglycols and amines, quaternized collagen polypeptides, polyethyleneimine, cationic silicone polymers, copolymers of adipic acid and dimethylaminohydroxypropyl diethylenetriamine, polyaminopolyamides, cationic chitin derivatives, cationic guar gum and/or quaternized ammonium salt polymers.

6. Cosmetic compositions as claimed in claims 1 to 5, characterized in that they contain the cationic polymers in quantities of 0.1 to 5% by weight.

## Revendications

1. Agents cosmétiques contenant :
a) des N-alkylpolyhydroxyalkylamides d'acide gras et,
b) des polymères cationiques
avec la restriction qu'ils sont dépourvus d'urée et de sel de monoéthanolamine et de 4-hydroxy-6-méthyl(2,4,4-triméthylpentyl)-2-pyridone.

2. Agents cosmétiques selon la revendication 1,
caractérisés en ce qu'
ils renferment des N-alkylpolyhydroxyalkylamides d'acide gras de formule (I) 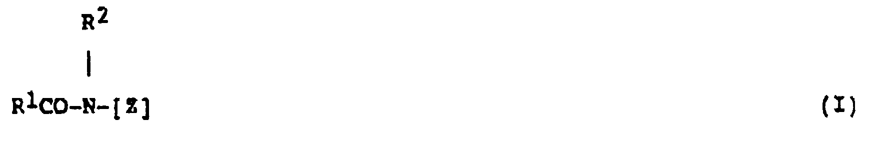 dans laquelle,
R¹CO représente un reste acyle aliphatique ayant de 6 à 22 atomes de carbone R² représente de l'hydrogène, un radical alkyle ou hydroxyalkyle, ayant de 1 à 4 atomes de carbone et,
[Z] représente un reste polyhydroxyalkyle linéaire ou ramifié, ayant de 3 à 12 atomes de carbone et de 3 à 10 groupes hydroxyle.

3. Agents cosmétiques selon les revendications 1 et 2,
caractérisés en ce qu'
ils renferment comme N-alkylpolyhydroxyalkylamide d'acide gras, un N-méthylglucamide d'acide gras.

4. Agents cosmétiques selon les revendications 1 et 2,
caractérisés en ce qu'
ils renferment les N-alkylpolyhydroxyalkylamides d'acide gras en quantités allant de 0,5 à 25 % en poids, rapporté à l'agent.

5. Agents cosmétiques selon les revendications 1 à 4,
caractérisés en ce qu'
ils contiennent des polymères cationiques choisis dans le groupe qui est formé par les dérivés de cellulose cationiques, les amidons cationiques, les copolymères de sels de diallylammonium et les acrylamides, les dérivés quaternisés de polyvinylpyrrolidone, les polymères quaternisés de vinylpyrrolidone/vinylimidazole, les produits de condensation de polyglycols et d'amines, les polypeptides quaternisés de collagène, la polyéthylèneimine, les polymères de silicone cationiques, les copolymères d'acide adipique et de diméthylaminohydroxypropyldiethylènetrimamine, les polyaminopolyamides, les dérivés cationiques de chitine, la gomme guar cationique et/ou les polymères de sel d'ammonium quaternisé.

6. Agents cosmétiques selon les revendications 1 à 5,
caractérisés en ce qu'
ils renferment les polymères cationiques en quantités allant de 0,1 à 5 % en poids.
